Europäisches Patentamt

European Patent Office

Office européen des brevets

⑱

(11) Publication number: **0 180 662**
**B1**

(12) **EUROPEAN PATENT SPECIFICATION**

(45) Date of publication of patent specification: **08.02.89**

(21) Application number: **84113482.8**

(22) Date of filing: **08.11.84**

(51) Int. Cl.⁴: **G 01 L 9/06,** A 61 B 5/02,
A 61 B 5/03

(54) Measuring transducer, in particular for medical applications.

(43) Date of publication of application:
**14.05.86 Bulletin 86/20**

(45) Publication of the grant of the patent:
**08.02.89 Bulletin 89/06**

(84) Designated Contracting States:
**BE CH DE FR GB IT LI NL**

(56) References cited:
**EP-A-0 124 308**
**WO-A-82/03684**
**US-A-3 592 187**
**US-A-4 416 156**

(73) Proprietor: **PPG HELLIGE B.V.**
**I.B.C.-Weg 1**
**NL-5683 PK Best (NL)**

(72) Inventor: **Kuypers, Martinus Henricus**
**Schoolstraat 53**
**NL-5561 AH Riethoven (NL)**

(74) Representative: **Patentanwälte TER MEER -**
**MÜLLER - STEINMEISTER**
**Mauerkircherstrasse 45**
**D-8000 München 80 (DE)**

**Description**

The invention relates to a measuring transducer which in particular is useful for medical applications such as extra corporeal measuring of pressure, oxygen content or other physical or chemical properties. A transducer of this type as described in the general portion of claim 1 is known from EP—A—0 124 308. The pressure sensor of this transducer is carried by an insulating body, which together with a first part of the hosing forms the fluid chamber between them. This insulating body also is provided with electrical pins by which the sensor can be connected to corresponding connecting means of an electrical connector plug held by the second part of the housing.

WO—A—8 203 684 shows a fluid pressure sensor with two pressure ports located aligned with respect to a common axis and a silicon sensor chip clamped between two portions of a housing and therewith being exposed with its two sides to the differential pressure between the two ports. There is no flow of fluid between the two ports.

It is the main object of the invention to disclose a disposable and inexpensive, but nevertheless, reliable and accurate transducer which can be reliably cleaned and pre-sterilized, can be easily attached to a fluid tubing, and can be used in connection with different diagnosis, patient monitoring or therapy equipment such as infusion apparatus, respirators or other equipment for medical care.

These objects are achieved by the invention as characterized in claim 1. Forming the housing of two parts supporting the sensor chip between them, facilitates sealing of the transducer and simultaneously provides mechanical protection of the sensitive transducer element or sensor against mechanical stress, in particular during transport, storage and installation. Preferred modifications and improvements are described in the subclaims. For a better understanding reference is made to the attached drawings in which two embodiments of the transducer are shown as follows:

Figure 1 shows a top view of the transducer and

Figure 2 a sectional view along Line II—II in Figure 1.

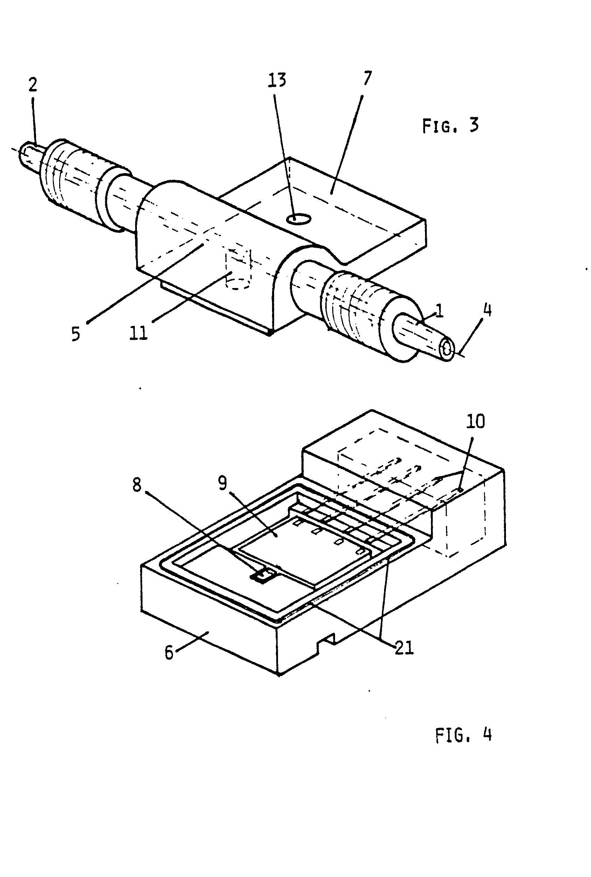

Figure 3 shows a perspective view of the upper part of the housing, and

Figure 4 the lower part of the housing with the sensor chip and adjusting circuitry inserted.

Figure 5 shows an enlarged sectional view through a first embodiment showing part of Figure 2 with the sensor chip and the means for sealing the two parts of the housing.

Figure 6 is an orthogonal view along sectional line VI—VI in Figure 5.

Figures 7 and 8 are a similar showing of a second embodiment.

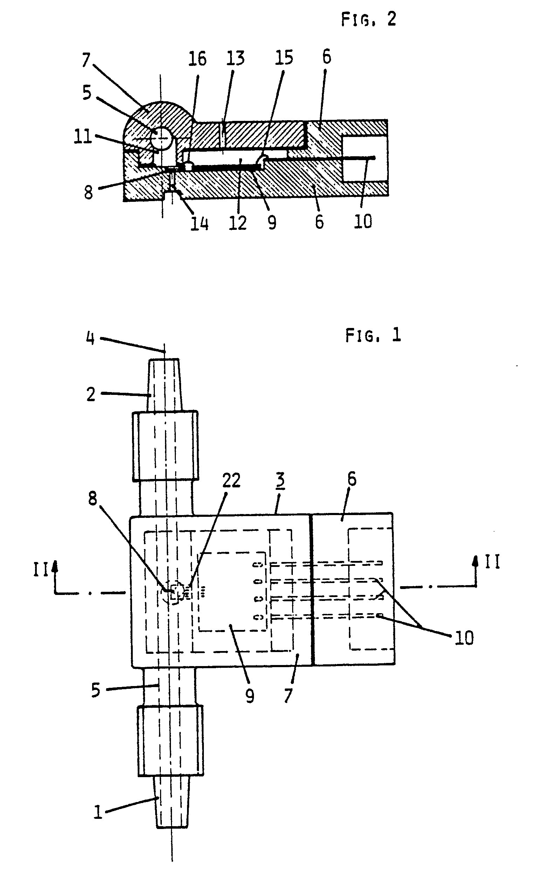

As can best be seen from Figure 1 the housing has a T-shaped configuration consisting of a fluid inlet 1, a fluid outlet 2 and a central box-like part 3 which extends rectangularly with respect to the common axis 4 of inlet 1 and outlet 2. Sensor housing 3 has a fluid passage 5 which is aligned with inlet 1 and outlet 2 forming a straightforward fluid channel through which the fluid under measurement can be passed. Sensor housing 3 consists (see Figure 2) of a first or bottom part 6 and a second or cover part 7. The first part 6 supports the sensor chip 8, a thin film or thick film substrate 9 and electrical connectors 10 which in the shown embodiment are pin connectors but could also be cable connectors or other electrical connecting means to external circuitry. The second part 7 of the housing is provided with the cylindrical fluid channel 5 extending along the same axis 4 as inlet 1 and outlet 2 and therewith forming a straight fluid passage. The second part 7 of the housing closes two chambers 11 and 12 formed between the two parts 6 and 7 of the housing. Chamber 11 communicates with passage 5 and in one of its wall portions the sensing portion of sensor chip 8 is exposed to the fluid entering inlet 1. The other chamber 12 is sealed against chamber 11 and contains the thin film or thick film substrate 9 which has provided thereon several adjusting resistors which can be trimmed with respect to their resistance either by laser trimming or sandblasting before the two parts of the housing are fixed together. Cover part 7 further has an opening 13 to the outside which may be used as a vent opening or as a filling hole for filling chamber 12 with epoxy resin or other electrically isolating fill protecting the adjusting resistors and the circuitry on film substrate 9, which may also include an amplifier.

If the sensor chip contains a solid state pressure sensor in the form of a silicon diaphragm with integrated strain-sensitive resistors, bottom portion 6 is provided with an opening 14 communicating with the bottom side of the sensing portion of sensor chip 8 and therewith forming a vent opening for the pressure sensor. Instead of electrical connectors 10 in the form of connector pins, a cable might be directly fixed, preferably cast to bottom part 6 of the housing and its wires cast into this bottom part. Connector pins 10 are electrically connected by wires 15 to the circuitry on film substrate 9. In the case of a cable being directly cast into housing part 6, the end portions of the cable wires can be soldered to substrate 9. Flexible connecting wires 16 provide electrical connection between the sensor chip 8 and the thin film or thick film substrate 9.

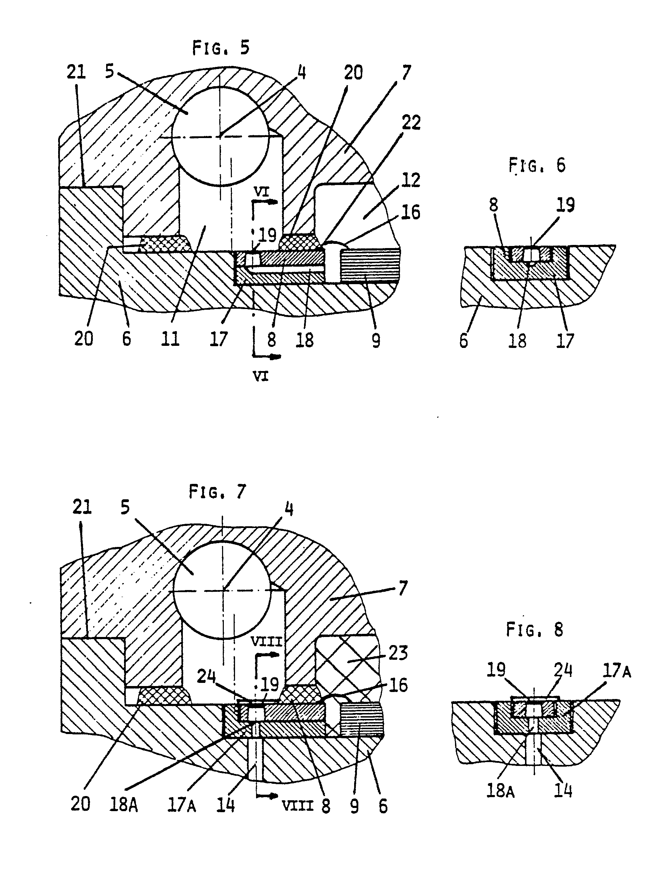

The configuration of the transducer permits to use the same housing for different types of sensors such as pressure sensor, oxygen sensor or sensors for other physical or chemical properties of a fluid flowing from inlet 1 to outlet 2 of the housing. When used for medical applications, a reliable electrical isolation between the electrical circuitry and the flowing fluid is an essential requirement. With reference to Figures 5 to 8 two embodiments of the detailed structure of the sensor chip, its supporting substrate and the electrical and fluid tight isolating means will be described.

For maintaining a good fluid tight and electrical isolation, it is important that in particular in case of a pressure sensing chip no mechanical stress at the border surfaces between the sensor chip and the housing is generated during operation. Such stress could be generated by changing temperatures or temperature differences or by mechanical forces. In order to prevent any mechanical influence of this kind acting upon the sensor chip and its condition sensitive portions, the sensor chip 8 as shown in Figure 5 to 8 is supported by a substrate 17 having about the same coefficient of thermal expansion as the sensor chip 8. As the sensor chip mainly consists of silicon having a linear expansion coefficient of $30 \times 10^{-7}$ mm/mm°C, this is substrate 17 preferably consists of silicon or FeNiCo ($50 \times 10^{-7}$ mm/mm°C) or Pyrexglass ($33 \times 10^{-7}$ mm/mm°C) or Ni-Fe 36 ($16 \times 10^{-7}$ mm/mm°C). Even a substrate made of stainless steel ($130 \times 107$ mm/mm°C). might be useful. The substrate can also consist of silicon or ceramics. If a metallic substrate is used, the corrosion durability of the metal can be improved by galvanic treatment and heating the substrate in reducing atmosphere. For fixing the sensor chip 8 to the substrate 16 suitable adhesives may be used or in the case of a pyrex glass substrate, the polished surfaces of the chip and of the pyrex glass substrate are brought into intimate contact by electrostatical forces at a temperature below the transformation temperature of the glass so that diffusion between the two parts will take place. Substrate 17 carries sensor chip 8 and in Figure 5 is provided with a passage 18 connecting the lower side of pressure-sensitive silicon diaphragm 19 to space 12. This space 12 via opening 13 (see Figure 2) communicates with the atmosphere. Instead of hole 13 a channel could be provided in parallel to connector pins 10. A fluid tight sealing between fluid chamber 11 and chamber 12 is achieved by inserting a silicon rubber ring 20 between those front surfaces of housing parts 6 and 7 which surround fluid chamber 11. Instead of a separate silicon rubber ring 20, a ring of silicon rubber adhesive or another type of adhesive may be used for achieving a fluid tight engagement between the two parts of the housing. In addition the engaging surfaces 21 of the two parts of the housing are either fixed to each other by an appropriate adhesive or by ultrasonic welding. The silicon rubber sheet or adhesive ring 20 provides good electrical isolation of the fluid against the electrical circuitry on film substrate 9 and the connections to the external circuitry. For connecting the sensor chip 8 to the circuitry on film substrate 9 connector pads 22 are provided on sensor chip 8 and are by means of flexible and therewith strain-free wires 16 electrically connected to the circuitry on film substrate 9.

In the second embodiment shown in Figures 7 and 8 the space 12 above film substrate 9 is filled with a casting resin 23, e.g. epoxy resin for protecting the resistors on substrate 9 and the associated circuitry and connecting wires. In order to expose the bottom side of pressure-sensitive diaphragm 19 to the atmosphere channel 18a in supporting substrate 17a in this case extends downward and ports into hole 14 extending through the base portion 6 of the housing. The surface of sensor chip 8 which is exposed to the fluid, i.e. in particular sensing portion 19, is covered by an electrically insulating layer 24 in order to maintain electrical safety of a patient during defibrillation or other electrical treatment. In case of a pressure sensor the venting channels 14, 18 can also be used for calibrating the sensor at different pressures and/or temperatures. The thick film or thin film resistors on film substrate 9 are used for balancing and calibrating the transducer and for this purpose can be changed with respect to their resistance value either by laser trimming or sandblasting.

If the sensor chip 8 is not responsive to pressure but to another physical or biological condition such as oxygen content, the linear expansion matched substrate 17 and the venting openings 14, 18 can be deleted. The sensor chip 8 then can merely be mounted in a trough-shaped portion of part 6 of the housing.

**Claims**

1. Measuring transducer, in particular for medical applications, comprising:
   a) a two-part housing (6, 7) with a fluid inlet (1), a fluid outlet (4) and a fluid chamber (11) between them;
   b) a solid state sensor chip (8) exposed to the fluid within said chamber and having electrical connecting means (22) for connecting the sensor chip (8) to an external circuitry, characterized in that:
   c) the fluid chamber (11) is formed between the two parts (6, 7) of the housing;
   d) the sensor chip (8) having a sensing portion (19) exposed to the fluid and another portion sealed agnst the fluid and provided with electrically conductive connector pads (22);
   e) the sensor chip (8) is located and fixed to the first part (6) of the housing;
   f) a sealing (20) seals the second part (7) of the housing against the first part (6) and against the sensor chip (8) as well, therewith forming the fluid chamber (11);
   g) electrical connectors (10) are provided in the first part (6) of the housing and are adapted to connect the connector pads (22) of the sensor chip (8) to the external circuitry.

2. Transducer according to claim 1, characterized in that both parts (6, 7) of the housing are fixed to each other and sealed against each other by means of an adhesive, preferably silicon rubber.

3. Transducer according to claim 1, characterized in that both parts (6, 7) of the housing are fixed to each other and sealed against each other by means of a sealing material (20), preferably silicon rubber, and by ultrasonic welding along the edges (21) of the two parts.

4. Transducer according to one of claims 1 to 3, characterized in that the first part (6) of the housing (6, 7) supports a thick film or thin film substrate (9) with adjusting resistors and/or signal converting circuitry thereon, whereat strain-free wire connections (16) are provided between the circuitry on said film substrate (9) and the connector pads (22) of the sensor chip (8).

5. Transducer according to claim 4, characterized in that the connector pads (22) are provided on a portion of the sensor chip (8) which extends into a space (12) of the housing in which the film substrate (9) is located.

6. Transducer according to claim 5, characterized in that the connector pads (22) and the film substrate (9) are covered by an electrically isolating layer.

7. Transducer according to claim 6, characterized in that the isolating layer is part of an epoxy resin fill (23) of said space (12).

8. Transducer according to one of claims 1 to 7, in particular pressure sensor comprising a solid state pressure sensor, characterized in that the sensor chip (8) is fixed to a substrate (17) having a coefficient of thermal expansion matched to the coefficient of the sensor chip and said substrate (17) is held in the first part (6) of the housing.

9. Transducer according to claim 8 with a pressure sensitive chip, characterized in that the sensing portion (19) of the sensor chip (8) is covered by an electrically insulating layer (24).

10. Transducer according to claims 8 or 9, characterized in that the sensor substrate (17) comprises a hole (18) or channel (18a) communicating with that side of the sensor chip (8) which is not exposed to the fluid.

11. Transducer according to claim 10, characterized in that the hole (18) or channel (18a) communicates with the ambient space outside the housing (6, 7).

12. Transducer according to one of claims 1 to 11, characterized in that the sensor substrate (17) consists of glass or ceramics.

13. Transducer according to one of claims 1 to 11, characterized in that a sensor substrate (17) consists of metal.

14. Transducer according to one of claims 1 to 13, characterized in that the inlet (1), the outlet (2) and a straight fluid channel (5) in the second part (7) of the housing are aligned along a common axis (4).

15. Transducer according to claim 4, characterized in that a box-shaped part of the housing (6, 7) enclosing the sensor chip (8), the film substrate (9) and the electrical connectors (10), projects away from said axis (4).

**Patentansprüche**

1. Meßwandler insbesondere für medizinische Anwendungen mit
a) einem zweiteiligen Gehäuse (6, 7) mit einem Fluideinlaß (1), einem Fluidauslaß (4) und einer dazwischen angeordneten Fluidkammer (11);
b) einem Festkörpersensorchip (8), das in der Kammer durch das Fluid beaufschlagt und mit elektrischen Anschlußelmenten (22) zum Anschluß des Sensorchips (8) an einen externen Schaltkreis versehen ist; dadurch gekennzeichnet, daß
c) die Fluidkammer (11) zwischen den beiden Teilen (6, 7) des Gehäuses ausgebildet ist;
d) das Sensorchip (8) einen Tastabschnitt (19), der dem Fluid ausgesetzt ist, und einen weiteren, gegen das Fluid abgedichteten Abschnitt aufweist, der mit elektrisch leitenden Anschlußpads (22) versehen ist;
e) das Sensorchip (8) im ersten Gehäuseteil (6) angeordnet und dort befestigt ist;
f) eine Abdichtung (20) die beiden Gehäuseteile (6 bzw. 7) gegeneinander und gegen das Sensorchip (8) abdichtet, um damit die Fluidkammer (11) zu umgrenzen;
g) elektrische Anschlüsse (10) im ersten Gehäuseteil (6) vorhanden sind zum Anschluß der Anschlußpads (22) des Sensorchips (8) an den äußeren Schaltkreis.

2. Meßwandler nach Anspruch 1, dadurch gekennzeichnet, daß beide Teile (6, 7) des Gehäuses mittels eines Klebemittels, vorzugsweise Siliconkautschuk, aneinander fixiert und gegeneinander abgedichtet sind.

3. Meßwandler nach Anspruch 1, dadurch gekennzeichnet, daß beide Teile (6, 7) des Gehäuses mittels eines Abdichtmaterials (20), vorzugsweise Siliconkautschuk, und durch Ultraschallverschweißen entlang der Kanten (21) der beiden Gehäuseteile miteinander verbunden und gegeneinander abgedichtet sind.

4. Meßwandler nach einem der Ansprüche 1 bis 3, dadurch gekennzeichnet, daß das erste Teil (6) des Gehäuses (6, 7) als Träger für ein Dickfilm- oder ein Dünnfilmsubstrat (9) mit Abgleichwiderständen und/oder einer Signalumsetzerschaltung dienen, an dem spannungsfrei geführte Anschlüsse (16) zwischen dem Schaltkreis auf dem Filmsubstrat (9) und den Anschlußpads (22) auf dem Sensorchip (8) vorgesehen sind.

5. Meßwandler nach Anspruch 4, dadurch gekennzeichnet, daß die Anschlußpads (22) an einem Bereich des Sensorchips (8) vorgesehen sind, der sich in einen Raum (12) des Gehäuses erstreckt, in dem das Filmsubstrat (9) angeordnet ist.

6. Meßwandler nach Anspruch 5, dadurch gekennzeichnet, daß die Anschlußpads (22) und das Filmsubstrat (9) durch eine elektrisch isolierende Schicht überdeckt sind.

7. Meßwandler nach Anspruch 6, dadurch gekennzeichnet, daß die isolierende Schicht Teil einer Epoxyharzfüllung (23) des erwähnten Raums (12) ist.

8. Meßwandler nach einem der Ansprüche 1 bis 7, insbesondere mit einem Festkörperdrucksensor, dadurch gekennzeichnet, daß das Sensorchip (8) auf einem Substrat (17) fixiert ist, dessen thermischer Ausdehnungskoeffizient an den entsprechenden Koeffizienten des Sensorchips angepaßt ist und daß das Substrat (17) im ersten Gehäuseteil (6) gehalten ist.

9. Meßwandler nach Anspruch 8, mit einem drucksensitiven Chip, dadurch gekennzeichnet, daß der Tastabschnitt (19) des Sensorchips (8) durch eine elektrisch isolierende Schicht (24) überdeckt ist.

10. Meßwandler nach Anspruch 8 oder 9, dadurch gekennzeichnet, daß das Sensorsubstrat (17) ein Loch (18) oder einen Kanal (18a) aufweist, der mit jener Seite des Sensorchips (8) kommuniziert, welche dem Fluid nicht ausgesetzt ist.

11. Meßwandler nach Anspruch 10, dadurch gekennzeichnet, daß das Loch (18) oder der Kanal (18a) mit der Umgebung außerhalb des Gehäuses (6, 7) kommuniziert.

12. Meßwandler nach einem der Ansprüche 1 bis 11, dadurch gekennzeichnet, daß das Sensorsubstrat (17) aus Glas oder Keramik besteht.

13. Meßwandler nach einem der Ansprüche 1 bis 11, dadurch gekennzeichnet, daß das Sensorsubstrat (17) aus Metall besteht.

14. Meßwandler nach einem der Ansprüche 1 bis 13, dadurch gekennzeichnet, daß der Einlaß (1), der Auslaß (2) und ein geradliniger Fluidkanal (5) im zweiten Gehäuseteil (7) entlang einer gemeinsamen Achse (4) ausgerichtet sind.

15. Meßwandler nach Anspruch 4, dadurch gekennzeichnet, daß sich ein schachtelartiger Teil des Gehäuses (6, 7), welcher as Sensorchip (8), das Filmsubstrat (9) und die elektrischen Anschlüsse (10) enthält, von der genannten Achse (4) weg erstreckt.

**Revendications**

1. Transducteur de mesure, en particulier pour applications médicales, comprenant
a) un boîtier en deux parties (6, 7) avec une entrée de fluide (1), une sortie de fluide (4) et une chambre de fluide (11) entre elles;
b) un capteur à pastille semi-conductrice (8) exposé au fluide dans ladite chambre et ayant des moyens de raccord électrique (22) pour raccorder la pastille de capteur (8) à un ensemble de circuits externes, caractérisé en ce que
c) la chambre de fluide (11) est formée entre les deux parties (6, 7) du boîtier;
d) la pastille de capteur (8) ayant une partie sensible (19) exposée au fluide et une autre partie protégée du fluide de façon étanche et munie de bornes de raccord conductrices de l'électricité (22);
e) la pastille de capteur (8) est située et fixée à la première partie (6) du boîtier;
f) un dispositif d'étanchéité (20) scelle la seconde partie (7) du boîtier contre le première partie (6) et contre la pastille de capteur (8) également, l'ensemble d'élimitant la chambre de fluide (11);
g) des raccords électriques (10) sont prévus dans la première partie (6) du boîtier et sont adaptés pour relier les bornes de raccord (22) de la pastille de capteur (8) à l'ensemble des circuits externes.

2. Transducteur selon la revendication 1, caractérisé en ce que les deux parties (6, 7) du boîtier sont fixées l'une à l'autre et scellées l'une contre l'autre au moyen d'un adhésif, de préférence un caoutchouc de silicone.

3. Transducteur selon la revendication 1, caractérisé en ce que les deux parties (6, 7) du boîtier sont fixées l'une à l'autre et scellées l'une contre l'autre au moyen d'une matière de scellement (20), de préférence un caoutchouc de silicone, et par soudage aux ultra-sons le long des bords (21) des deux parties.

4. Transducteur selon l'une des revendications 1 à 3, caractérisé en ce que la première partie (6) du boîtier (6, 7) porte un substrat sous forme de film épais ou de film mince (9) sur lequel se trouvent des résistors ajustables et/ou un ensemble de circuits de transformation de signaux, où des fils de raccords sans contrainte (16) sont prévus entre l'ensemble de circuits sur ledit substrat sous forme de film (9) et les bornes de raccord (22) de la pastille de capteur (8).

5. Transducteur selon la revendication 4, caractérisé en ce que les bornes de raccord (22) sont prévues sur une partie de la pastille de capteur (8) qui s'étend dans un espace (12) du boîtier dans lequel le substrat sous forme de film (9) est situé.

6. Transducteur selon la revendication 5, caractérisé en ce que les bornes de raccord (22) et le substrat sous forme de film (9) sont recouverts par une couche électriquement isolante.

7. Transducteur selon la revendication 6, caractérisé en ce que la couche isolante constitue une partie d'un remplissage de résine époxy (23) dudit espace (12).

8. Transducteur selon l'une des revendications 1 à 7, en particulier capteur de pression comprenant un capteur de pression à semi-conducteur, caractérisé en ce que la pastille de capteur (8) est fixée à un substrat (17) ayant un coefficient de dilatation thermique correspondant au coefficient de la pastille de capteur et en ce que ledit substrat (17) est maintenu dans la première partie (6) du boîtier.

9. Transducteur selon la revendication 8 avec une pastille sensible à la pression, caractérisé en ce que la partie sensible (19) de la pastille de capteur (8) est recouverte d'une couche électriquement isolante (24).

10. Transducteur selon les revendications 8 ou 9, caractérisé en ce que le substrat du capteur (17) comprend un trou (18) ou cheminement (18a) communiquant avec le côté de la pastille de capteur (8) qui n'est pas exposé au fluide.

11. Transducteur selon la revendication 10, caractérisé en ce que le trou (18) ou cheminement (18a) communique avec l'espace ambiant à l'extérieur du boîtier (6, 7).

12. Transducteur selon l'une des revendications 1 à 11, caractérisé en ce que le substrat du capteur (17) est en verre ou en céramique.

13. Transducteur selon l'une des revendications 1 à 11, caractérisé en ce qu'un substrat de capteur (17) est en métal.

14. Transducteur selon l'une des revendications 1 à 13, caractérisé en ce que l'entrée (1), la sortie (2) et un cheminement de fluide droit (5) dans la seconde partie (7) du boîtier sont alignés le long d'un axe commun (4).

15. Transducteur selon la revendication 4, caractérisé en ce qu'une partie en forme de boîte du boîtier (6, 7) renfermant la pastille de capteur (8), le substrat sous forme de film (9) et les raccords électriques (10), fait saillie au-delà dudit axe (4).

FIG. 2

FIG. 1

EP 0 180 662 B1

FIG. 3

FIG. 4

2

FIG. 5

FIG. 6

FIG. 7

FIG. 8